# EUROPÄISCHE PATENTSCHRIFT

(11) **EP 2 359 889 B1**
(45) Veröffentlichungstag und Bekanntmachung des Hinweises auf die Patenterteilung: **05.06.2013**
(21) Anmeldenummer: 10154573.9
(22) Anmeldetag: 24.02.2010
(51) Int. Cl.: A61M 16/08, A61B 5/097

(54) **Vorrichtung zur Entnahme einer Atemgasprobe**
Device for obtaining a breathing gas sample
Dispositif de prélèvement d'un échantillon de gaz respiratoire

(43) Veröffentlichungstag der Anmeldung: 24.08.2011
(73) Patentinhaber: Dräger Medical GmbH, 23558 Lübeck (DE)
(72) Erfinder: Heesch, Ralf, 23568, Lübeck (DE)
(74) Vertreter: UEXKÜLL & STOLBERG

(56) Entgegenhaltungen:
- EP-A1- 0 827 713
- DE-A1- 19 939 479
- US-A- 5 213 096
- US-A1- 2004 065 329
- US-A1- 2005 257 791
- US-B1- 6 935 338

## Beschreibung

Die Erfindung betrifft eine Vorrichtung zur Entnahme einer Atemgasprobe zur Bestimmung von Bestandteilen in Atemgasen.

Aus dem Stand der Technik sind Messungen zur Bestimmung von Bestandteilen in Atemgasen, insbesondere bei der künstlichen Patientenbeatmung bekannt. Die Messergebnisse werden für eine Analyse der Funktion des Atemapparates und zur Erhaltung weiterer Körperfunktionen des Patienten verwendet. Hierbei wird zwischen absaugenden Verfahren und der Messung im Hauptstrom des Atemgases unterschieden. Die vorliegende Erfindung bezieht sich auf ein absaugendes Verfahren.

Bei einem absaugenden Verfahren wird mittels einer Pumpe kontinuierlich eine Teilmenge des Atemgases entnommen. Diese wird vorzugsweise von einem Tubusanschlussstück abgesaugt und mittels einer Probengasleitung an eine Messvorrichtung für eine Atemgasanalyse geführt. Mit der Messvorrichtung wird sowohl die inspiratorische als auch die expiratorische Atemgaskonzentration analysiert.

Im Stand der Technik ist das Tubusanschlussstück mit einer Öffnung versehen, durch die eine entsprechende Atemgasprobe zu der Messeinrichtung abgesaugt werden kann. Nachteilig erweist sich hierbei eine Vermischung des inspiratorischen Atemgases mit dem expiratorischen Atemgas innerhalb des Tubusanschlussstückes. Insbesondere eine Messung einer CO₂ - Konzentration im expiratorischen Atemgas ist für die Ermittlung und Triggerung verschiedener physiologischer Parameter von großem Interesse. Eine Entnahme einer Messprobe aus der expiratorischen Atemgasströmung nahe der Lunge wäre hierbei von großem Vorteil.

Dokument US 5,213,096 offenbart ein Tubusanschlussstück für eine Vorrichtung zur Bestimmung von Bestandteilen in Atemgasen mit einem Anschluss an ein Beatmungssystem, einem Tubusanschluss und einer Wandöffnung, durch die sich ein Entnahmerohr zur Entnahme von Atemproben in Richtung des Tubusanschlusses erstreckt. Das Entnahmerohr ist vorgesehen, um mit einer Messvorrichtung verbunden zu werden und weist an seinem proximalen Ende mehrere seitlich ausgebildete Stege auf.

Dokument US 6,935,338 B1 beschreibt eine Vorrichtung zur Bestimmung von Bestandteilen in Atemgasen mit einer Messvorrichtung und mit einem Tubusanschlussstück, das eine Wandöffnung, einen Anschluss an ein Patientenbeatmungssystem, einen Tubusanschluss und ein sich durch die Wandöffnung erstreckendes Entnahmerohr aufweist. Das Entnahmerohr weist zwei gegenüberliegende seitliche Aussparungen auf, so dass ein Atemgasfluss senkrecht zu einer Atemgasentnahmerichtung durch die Aussparungen ermöglicht ist.

Dokument EP 0 827 713 A1 offenbart ein Tubusanschlussstück mit einem Anschluss an ein Patientenbeatmungssystem, einem Tubusanschluss und einer Wandöffnung, durch die sich ein Entnahmerohr in Richtung des Tubusanschlusses mit mehreren seitlichen Öffnungen erstreckt.

Der Erfindung liegt somit die Aufgabe zugrunde, eine Vorrichtung zur Bestimmung von Bestandteilen in Atemgasen derart zu verbessern, dass die Konzentrationsbestandteile im Atemgas, insbesondere im expiratorischen Atemgas zuverlässig bestimmbar sind.

Diese Aufgabe wird durch eine Vorrichtung mit den Merkmalen des unabhängigen Patentanspruchs gelöst.

In den davon abhängigen Patentansprüchen sind vorteilhafte Ausgestaltungen und Weiterbildungen der Erfindung angegeben.

Die Lösung der Aufgabe erfolgt dadurch, dass die Vorrichtung zur Entnahme einer Atemgasprobe zur, Bestimmung von Bestandteilen in Atemgasen als eine Tubusanschlussstück mit einer Wandöffnung, einem Anschluss an ein Patientenbeatmungssystem und einem Tubusanschluss ausgeführt ist, wobei ein von der Wandöffnung in Richtung des Tubusanschlusses verlaufendes Entnahmerohr vorgesehen ist, dessen proximales Ende wenigstens eine Aussparung aufweist, so dass ein Atemgasfluss senkrecht zur Gasentnahmerichtung ermöglicht ist.

In vorteilhafter Weise wird dadurch die Atemgasprobe direkt im Atemgasstrom unmittelbar vor einer Tubusöffnung eines mit der Vorrichtung verbundenen Tubus entnommen. Eine Entnahme einer Atemgasprobe für eine Analyse kann somit unmittelbar hinter der Mundöffnung eines Patienten erfolgen, so dass präzisere Angaben über die Zusammensetzung und die Konzentration der Atemgase in der Lunge erzielt werden können.

Eine Veränderung der Atemgaskonzentration auf dem Weg zum Messort kann damit in vorteilhafter Weise reduziert werden. Mit der erfindungsgemäßen Vorrichtung kann ferner eine verbesserte Signalflanke einer CO₂-Konzentration im expiratorischen Atemgas erreicht werden. Die entnommene Atemgasprobe kann dadurch schnell und ohne weitere Vermischung in der Vorrichtung, insbesondere in einem Tubusanschlussstück an den Messsensor gelangen. Die Zuverlässigkeit und die Genauigkeit der Messergebnisse lassen sich somit verbessern.

Die erfindungsgemäße Vorrichtung erweist sich durch eine genauere Bestimmung des CO₂-Wertes im expiratorischen Atemgas und einer damit einhergehenden besseren Auflösung der Signalflanke für Patienten mit einer hohen Beatmungsfrequenz und einer damit einhergehenden schnelleren CO₂-Signalflankensteuerung als besonders vorteilhaft. Insbesondere gilt dies für Patienten mit einem geringeren Atemgasvolumen.

Mit der erfindungsgemäßen Vorrichtung lässt sich ferner die Sicherheit in der Patientenbeatmung insbesondere bei der Beatmung neonatalen Patienten verbessern.

In einer ersten vorteilhaften Ausführung der erfindungsgemäßen Vorrichtung ist das Entnahmerohr zentrisch in der Achse des Tubusanschlusses angeordnet.

In einer weiteren vorteilhaften Ausbildung ist ein Außenmaß des Entnahmerohres größer als ein Durchmesser der Öffnung eines Tubus. Alternativ hierzu kann das proximale Ende des Entnahmerohres derart ausgebildet sein, dass bei einem Zusammenwirken des Entnahmerohrs mit dem Tubus die Öffnung des Tubus um maximal 30 % verringert wird. Ein ausreichender Atemgasstrom zur künstlichen Beatmung eines Patienten ist damit gewährleistet. Eine vorteilhafte Ausbildung der erfindungsgemäßen Vorrichtung hierzu ist, das proximale Ende des Entnahmerohrs mit wenigstens einem seitlichen Steg auszuführen. Der wenigstens eine Steg ist in radialer Richtung von der axialen Mitte des Entnahmerohrs ausgebildet.

Eine weitere vorteilhafte Ausbildung der erfindungsgemäßen Vorrichtung wäre eine Gestaltung des proximalen Endes des Entnahmerohrs mit einem dreieckigen Querschnitt mit einem in dessen Mitte zylinderförmigen Kanal.

In einer weiteren Ausführung kann der Querschnitt des proximalen Endes des Entnahmerohrs mit wenigstens zwei radial symmetrisch angeordneten Kerben ausgeführt sein.

Eine Unterbrechung des Atemgasstromes bei einem Zusammenwirken des Entnahmerohres mit dem Tubus wird in den vorstehenden Varianten somit in vorteilhafter Weise vermieden.

Eine weitere Ausführung der erfindungsgemäßen Vorrichtung besteht in axialer Anordnung des Entnahmerohres zu dem Tubusanschluss. Alternativ hierzu kann das Entnahmerohr in einem Winkel von 45° zu der axialen Richtung des Tubusanschlusses angeordnet sein. Es ist auch denkbar, das Entnahmerohr in weiteren Winkelpositionen zu der axialen Richtung des Tubusanschlusses anzuordnen.

In vorteilhafter Weise ist wenigstens ein Teil des Entnahmerohrs aus einem elastischen Material ausgeführt.

Alternativ hierzu kann das Entnahmerohr beweglich in der Wand des Tubusanschlussstücks befestigt sein oder mit einer elastischen Membran in der Wand des Tubusanschlussstücks fixiert sein.

Die erfindungsgemäße Vorrichtung kann Verwendung für die künstliche Beatmung von Patienten finden.

Die vorliegende Erfindung wird mit Bezug auf die beigefügten Zeichnungen detailliert erläutert, wobei gleiche Bezugszeichen gleiche Figuren bezeichnen.

In der Zeichnung gilt:
- Fig. 1: schematische Darstellung einer Vorrichtung zur Bestimmung von Bestandteilen in Atemgasen aus dem Stand der Technik,
- Fig. 2: schematische Darstellung einer Seitenansicht der erfindungsgemäßen Vorrichtung in der Ausführung eines Tubusanschlussstückes,
- Fig. 3: schematische Schnittdarstellung der erfindungsgemäßen Vorrichtung gemäß der Schnittlinie dargestellt in Fig. 2,
- Fig. 4: schematische Darstellung von drei Varianten eines Entnahmerohrs mit den jeweiligen Schnittbildern,
- Fig. 5: Schnittdarstellung der erfindungsgemäßen Vorrichtung in einer Ausführung mit Aussparungen in axialer Richtung des Entnahmerohres und einem seitlich ausgebildeten Steg,
- Fig. 6: schematische Darstellung der erfindungsgemäßen Vorrichtung mit einem Entnahmerohrs aufweisend einen elastischen Balg,
- Fig. 7: schematische Darstellung der erfindungsgemäßen Vorrichtung mit einem elastisch in der Wand gelagerten Entnahmerohr,
- Fig. 8: schematische Darstellung der erfindungsgemäßen Vorrichtung mit einem federnd gelagerten Entnahmerohr und
- Fig. 9: schematische Darstellung der erfindungsgemäßen Vorrichtung mit einem schräg angeordneten Entnahmerohr.

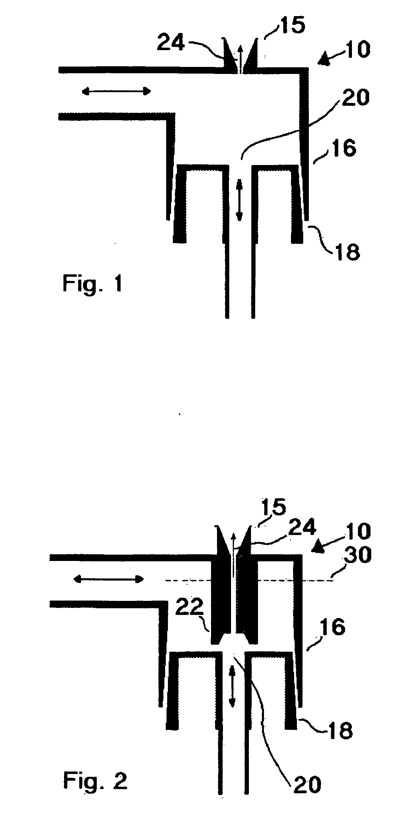

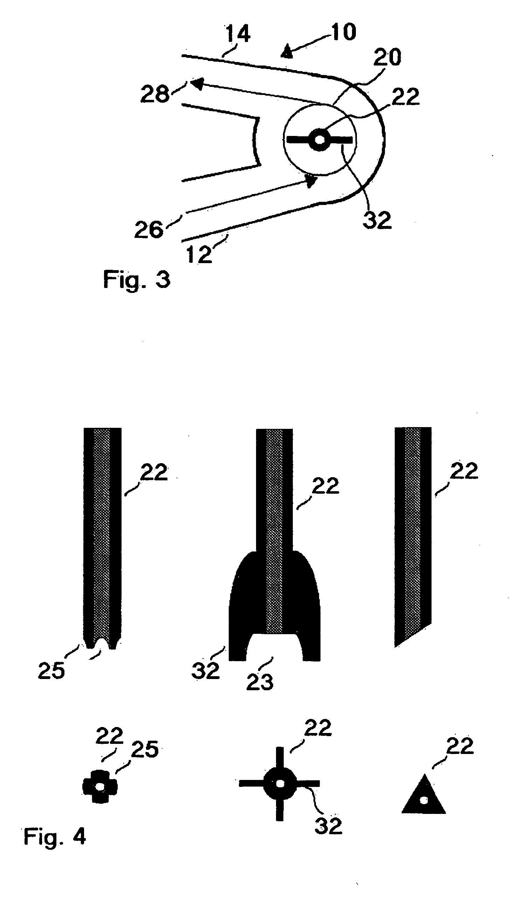

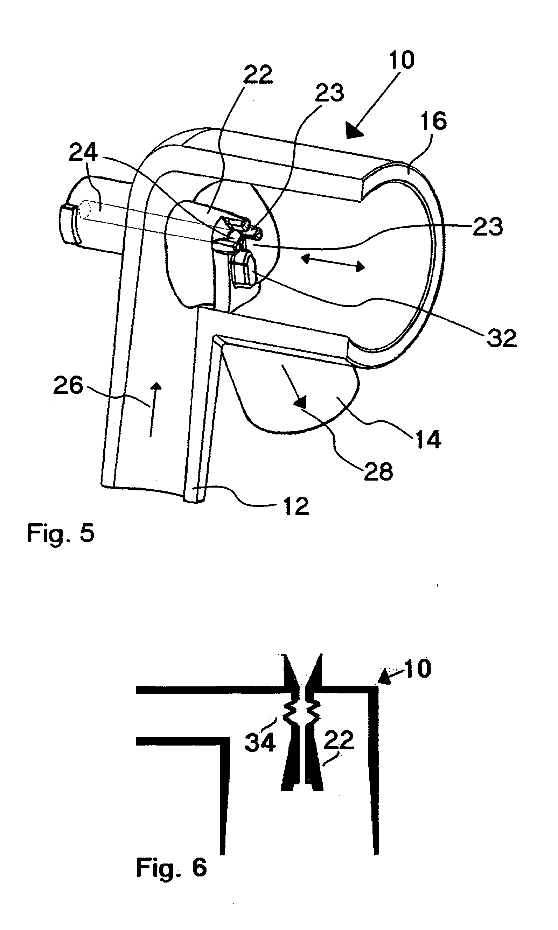

In Fig. 1 ist eine aus dem Stand der Technik bekannte Vorrichtung zur Bestimmung von Bestandteilen in Atemgasen als ein Tubusanschlussstück 10 dargestellt. Das Tubusanschlussstück 10 ist in der Form eines Y-Stückes ausgebildet. Das Tubusanschlussstück 10 weist einen Anschluss 15 für die Entnahme einer Atemgasprobe zur Bestimmung von Bestandteilen in Atemgasen auf. Hierzu ist das Tubusanschlussstück 10 mit einer Wandöffnung 24 ausgeführt, durch die die Atemgasprobe mittels einer Pumpe (nicht dargestellt) kontinuierlich dem Atemgasstrom entnommen wird. An dem Anschluss 15 zur Entnahme der Atemgasprobe ist ein Schlauch angeschlossen, der die Atemgasprobe an eine Messvorrichtung weiterleitet (nicht dargestellt). Das Tubusanschlussstück 10 weist ferner Anschlüsse 12 und 14 an ein Patientenbeatmungssystem auf (dargestellt in den Figuren 3 und 5). Das Tubusanschlussstück 10 wird an das Patientenbeatmungssystem vorzugsweise mittels Inspirations- und Expirationsschlauch angeschlossen. Der Inspirationszweig und der Expirationszweig sind in Form einer geschlossenen Ringleitung miteinander verbunden und werden an dem Tubusanschlussstück 10 zusammengeführt.

Das Tubusanschlussstück 10 enthält weiter einen Tubusanschluss 16 zur Verbindung des Tubusanschlussstücks 10 mit einem Tubus 18. Der Patient wird über den Tubus 18 an das Patientenbeatmungssystem angeschlossen. Ein Gebläse als Atemgasfördereinheit bewirkt eine Zirkulation des Atemgases in dem Atemsystem (nicht dargestellt). Zur Steuerung der inspiratorischen und expiratorischen Atemphase ist in dem Inspirationszweig ein ansteuerbares Inspirationsventil und in dem Expirationszweig ein ansteuerbares Expirationsventil enthalten (nicht dargestellt). Bei geöffnetem Inspirationsventil und geschlossenem Expirationsventil baut sich in der Lunge des Patienten ein Inspirationsdruck auf. Demgegenüber wird bei einem geschlossenen Inspirationsventil und einem geöffnetem Expirationsventil über eine Ansaugung der Atemgasfördereinheit die Lunge des Patienten entleert. Über den Anschluss 15 für die Entnahme einer Atemgasprobe werden die inspiratorischen als auch die expiratorischen Werte im Atemgasstrom gemessen.

In Fig. 2 ist schematisch eine seitliche Ansicht der erfindungsgemäßen Vorrichtung in der Ausführung eines Tubusanschlussstückes 10 mit einem Anschluss 15 für die Entnahme einer Atemgasprobe für eine Bestimmung von Bestandteilen in dem Atemgas dargestellt. Das Tubusanschlussstück 10 umfasst wiederum einen Anschluss für einen Inspirationsschlauch und einen Anschluss für einen Expirationsschlauch. Ferner weist das Tubusanschlussstück 10 einen rechtwinklig zu den genannten Anschlüssen angeordneten Tubusanschluss 16 auf.

Von einer Wandöffnung 24 des Tubusanschlussstücks 10 führt ein Entnahmerohr 22 in Richtung des Tubusanschlusses 16. Um eine Beschreibung der vorliegenden Erfindung zu ermöglichen, soll das in das Tubusanschlussstück hineinragende Ende des Entnahmerohrs 22 als das proximale Ende bezeichnet werden und das aus dem Tubusanschlussstück herausragende Ende des Entnahmerohrs 22 als das distale Ende. Das proximale Ende des Entnahmerohrs 22 ist gegenüber einer Tubusöffnung 20 angeordnet. Ein Tubus 18 kann an dem Tubusanschluss 16 mit dem Tubusanschlussstück 10 verbunden werden. Gehalten wird der Tubus 18 an dem Tubusanschluss 16 durch eine konusförmige Verbindung. Durch die Tubusöffnung 20 des Tubus 18 strömt der inspiratorische und expiratorische Atemgasstrom zum und vom Patienten.

Das Außenmaß des Entnahmerohrs 22 ist größer ausgeführt als der Durchmesser der Tubusöffnung 20. Das Entnahmerohr 22 weist an seinem distalen Ende eine Anschlussmöglichkeit für einen Schlauch zur Absaugung einer Atemgasprobe an ein Messsystem auf. Die Verbindung des Schlauches mit dem Entnahmerohrs 22 erfolgt üblicherweise mit einer sogenannten Luer-Verbindung.

Die vorteilhafte Ausbildung des Tubusanschlussstückes 10 ermöglicht eine Probenentnahme in unmittelbarer Nähe der Tubusöffnung 20. Damit können die Signalflanken des in dem expiratorischen Atemgas enthaltenen Kohlendioxides optimal ermittelt werden. Die proximale Öffnung des Entnahmerohrs 22 befindet sich in dem Atemgasstrom in unmittelbarer Nähe der Patientenlunge, so dass die aus der Patientenlunge abgeatmete Atemluft ohne Verwirbelungen und Vermischungen an das Messsystem (nicht dargestellt) weitergeleitet werden kann.

Fig. 3 zeigt einen Querschnitt des in der Fig. 2 dargestellten Tubusanschlussstücks 10 an einer Schnittlinie 30. Die Strömungsrichtungen des Atemvolumens sind entsprechend mit Pfeilen gekennzeichnet. In der Richtung des Inspirationsstromes 26 im Tubusanschlussstück 10 befindet sich ein Anschluss 12 für einen Inspirationsschlauch, wohingegen in der Richtung des Expirationsstromes 28 ein Anschluss 14 für einen Expirationsschlauch vorgesehen ist.

Der Tubus 18 ist mit einer konischen Verbindungsfläche zum Anschluss an das Tubusanschlussstück 10 ausgeführt. Insbesondere in der Ausführung zum Einmalgebrauch können sowohl die Außenmaße des Tubus 18 als auch die konischen Verbindungsflächen Maßtoleranzen aufweisen. Dadurch kann es zu einem Zusammenschluss von dem Tubus 18 insbesondere der Tubusöffnung 20 mit dem Entnahmerohr 22 kommen. Ein Blockieren des Atemgasstromes könnte die Folge sein, was zu einer Entkopplung des Patienten vom Beatmungskreis führen würde. Zudem wird mit einer absaugenden Probennahme des Atemgases das komplette Lungenvolumen von ca. 200 ml / min entnommen. Diese Entnahme kann bei einer Blockierung des Atemgasstromes in Folge zu einem negativen Druck in der Patientenlunge führen. Das Entnahmerohr 22 ist in vorteilhafter Weise an dem proximalen Ende mit zwei radial verlaufenden Stegen 32 ausgeführt. Mittels der Stege 32 wird ein Außenmaß des Entnahmerohrs 10 an dessen proximalen Ende erhöht und gegenüber dem Durchmesser der Tubusöffnung 20 vergrößert. Damit wird sichergestellt, dass bei einer Verbindung des Tubus 18 an dem Tubusanschluss 16 des Tubusanschlussstücks 10 ein Atemgasstrom durch das proximale Ende des Entnahmerohrs 22 nicht blockiert wird.

Ferner ist aus dem Schnittbild der Figur 3 die optimale Positionierung der Stege 32 in Bezug auf die in dem Tubusanschlussstück 10 vorherrschenden Strömungsrichtung der Atemgase gezeigt. Sowohl der inspiratorische Atemgasstrom 26 als auch der expiratorische Atemgasstrom 28 verlaufen nahezu parallel zu den Stegen 32, wodurch sich ein geringer Atemwegswiderstand für den Patienten ergibt.

Für die Durchführung einer sicheren Patientenbeatmung weisen die Stege 32 eine geringe Tiefe auf. Vorzugsweise entspricht die Tiefe drei Millimetern. In dieser konstruktiven Ausführung der erfindungsgemäßen Vorrichtung ist sichergestellt, dass ein Patient bei einer künstlichen Beatmung durch ein Zusammenwirken von dem Entnahmerohr 22 mit der Tubusöffnung 20 nicht von dem Beatmungskreis entkoppelt wird.

In der Darstellung der Figur 3 ist weiter eine aktive Fläche für die Atemgasströmung im Bereich der Tubusöffnung 20 in Zusammenwirkung mit dem Entnahmerohr 22 und dessen seitlichen Stegen 32 sichtbar, die sich beim Eingreifen des Entnahmerohrs 22 in die Tubusöffnung 20 ergibt. Konstruktiv sind die radial an der proximalen Öffnung des Entnahmerohrs 22 angeordneten Stege 32 so ausgeführt, dass bei einem Zusammenwirken des Entnahmerohrs 22 mit dem Tubus 18 die Tubusöffnung 20 um maximal 30 % verringert wird. Somit ergibt sich eine minimale aktive Strömungsfläche für einen Atemgasaustausch von 70 % der Tubusöffnung 20.

In der Fig. 4 sind hierzu schematisch und beispielhaft drei Varianten des proximalen Endes des Entnahmerohrs 22 mit den zugehörigen Schnittbildern dargestellt.

In der linken Ausführungsvariante des Entnahmerohrs 22 ist das proximale Ende mit vier radial symmetrisch angeordneten Kerben 25 ausgeführt. Die mittlere Ausführungsvariante des Entnahmerohrs 22 weist im proximalen Ende vier symmetrisch angeordneten Stegen 32 auf. Alternativ hierzu können auch jeweils ein bis drei Stege an dem proximalen Ende des Entnahmerohrs 22 vorgesehen sein. Die rechte Ausführungsvariante des Entnahmerohrs 22 zeigt einen dreieckförmigen Querschnitt.

Die in der Fig. 4 dargestellten Varianten der Ausgestaltung des proximalen Endes des Entnahmerohrs 22 stellen sicher, dass kein Patient durch ein Zusammenwirken von dem Entnahmerohrs 22 mit der Tubusöffnung 20 des Tubus 18 geschädigt wird.

Die Figur 5 zeigt eine Schnittdarstellung der erfindungsgemäßen Vorrichtung in der Form eines Tubusanschlussstücks 10 mit einem Entnahmerohr 22 aufweisend drei Aussparungen 23. Die Aussparungen 23 sind in axialer Richtung des Entnahmerohres 22 ausgebildet. Zusätzlich weist das proximale Ende des Entnahmerohres 22 einen seitlich ausgebildeten Steg 32 auf. Sowohl das Entnahmerohr 22 als auch die drei Aussparungen 23 und der Steg 32 können einteilig mit dem Tubusanschlussstücks 10 ausgebildet sein. Vorzugsweise wird das Tubusanschlussstücks 10 im Spritzgussverfahren hergestellt. Alternativ hierzu kann das Entnahmerohres 22 mit den drei Aussparungen 23 und das Tubusanschlussstück 10 jeweils einteilig ausgebildet sein. Der Steg 32 ist vorzugsweise in dieser Ausbildung an der inneren Wand des Tubusanschlussstücks 10 vorgesehen.

In den Figuren 6 bis 9 werden weitere vorteilhafte Ausbildungen der erfindungsgemäßen Vorrichtung gezeigt.

Dabei ist in Figur 6 wenigstens ein Teil des Entnahmerohrs 22 aus einem elastischen Material ausgeführt. Ein der Wandung eines Tubusanschlussstücks 10 zugewandter Teil des Entnahmerohrs 22 ist dabei als eine Art elastischer Balg 34 aufgebaut, der bei einem Zusammenwirken des Entnahmerohrs 22 mit dem Tubus 18 elastisch komprimiert werden kann. Somit wird ein Ausweichen des Entnahmerohrs 22 bei Einbringen des Tubus 18 in die Tubusöffnung 20 ermöglicht.

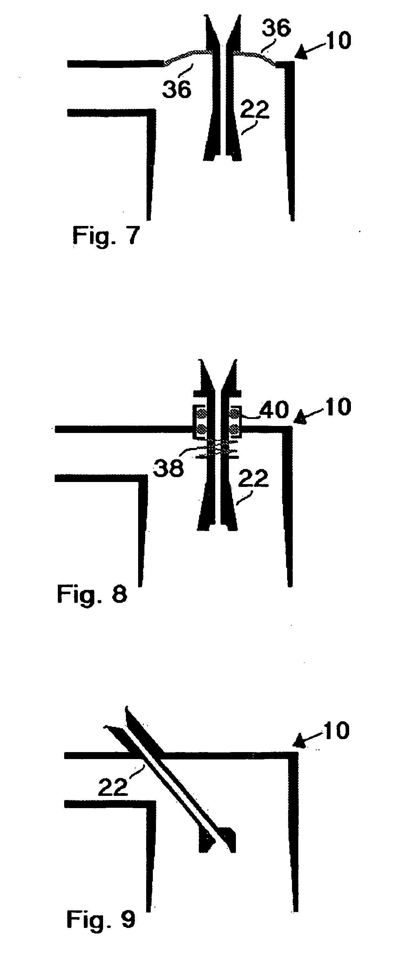

In der in Figur 7 dargestellten Ausführung ist das Entnahmerohr 22 in einem Tubusanschlussstück 10 innerhalb einer elastischen Membran 36 gelagert. Damit ist das Entnahmerohr 22 bei einer Verbindung des Tubus 18 mit dem Tubusanschlussstück 10 und einem Zusammenwirken mit dem Tubus 18 verschiebbar. Die elastische Membran 36 ist in vorteilhafter Weise so ausgelegt, dass diese nicht schon bei Beatmungsdrücken von ca. 80 hPa eine signifikante Auslenkung erfährt. Die elastische Membran 36 kann als separates Teil in das Tubusanschlussstück 10 eingebettet werden oder in einem Zwei-Komponenten-Spritzgussverfahren direkt mit dem Tubusanschlussstück 10 gefertigt werden.

Eine weitere Ausführung ist in der Figur 8 gezeigt, bei der das Entnahmerohr 22 in einer mit wenigstens einem O-Ring 40 pneumatisch dichtende Führung gelagert ist. Ein Federelement 38 ermöglicht hier eine axiale Verschiebung des Entnahmerohrs 22.

In der in Figur 9 dargestellten Ausführung ist das Entnahmerohr 22 in dem Tubusanschlussstück 10 in einem Winkel von ca. 45° zu der axialen Richtung der Tubusöffnung 20 angeordnet. Das Entnahmerohr 22 ist ferner aus einem elastischen Material vorzugsweise aus einem Gummi ausgeführt. Damit wird bei einem Zusammenwirken mit dem Tubus 18 eine Verschiebung des Entnahmerohrs 22 ermöglicht. Aufgrund der elastischen Eigenschaft des Entnahmerohrs 22 kann dieses selbsttätig bei einer Entkopplung des Tubus 18 von dem Tubusanschlussstück 10 in seine Ausgangsposition zurückgelangen.

Während die vorliegende Erfindung unter Bezugnahme auf die bevorzugten Ausführungsbeispiele beschrieben worden ist, sind dem Fachmann verschiedene Änderungen und Modifikationen klar. All diese Änderungen und Modifikationen sollen in den Schutzbereich der angeführten Ansprüche fallen.

### BEZUGSZEICHENLISTE

| | |
|---|---|
| 10 - | Tubusanschlussstück |
| 12 - | Anschluss für einen Inspirationsschlauch |
| 14 - | Anschluss für einen Expirationsschlauch |
| 15 - | Atemgasprobenentnahme |
| 16 - | Tubusanschluss |
| 18 - | Tubus |
| 20 - | Tubusöffnung |
| 22 - | Entnahmerohr |
| 23 - | Aussparung |
| 24 - | Wandöffnung |
| 25 - | Kerbe |
| 26 - | Inspirationsstrom |
| 28 - | Expirationsstrom |
| 30 - | Schnittlinie |
| 32 - | Stege |
| 34 - | Balg |
| 36 - | Membran |
| 38 - | Federelement |
| 40 - | O-Ring |

## Patentansprüche

1. Vorrichtung zur Entnahme einer Atemgasprobe aufweisend wenigstens
ein Tubusanschlussstück (10) mit einer Wandöffnung (24), einem Anschluss (12, 14) an ein Patientenbeatmungssystem, einem Tubusanschluss (16) und mit einem von der Wandöffnung (24) in Richtung des Tubusanschlusses (16) verlaufenden Entnahmerohr (22),
**dadurch gekennzeichnet,**
**dass** das proximale Ende des Entnahmerohrs (22) wenigstens eine Aussparung (23) aufweist, so dass ein Atemgasfluss senkrecht zu einer Atemgasentnahmerichtung ermöglicht ist.

2. Vorrichtung nach Anspruch 1, **dadurch gekennzeichnet, dass** das Entnahmerohr (22) so ausgebildet ist, dass es bei Anwendung zentrisch in der Achse der Tubusöffnung (20) angeordnet ist.

3. Vorrichtung nach Anspruch 1, **dadurch gekennzeichnet, dass** das Entnahmerohr (22) so ausgebildet ist, dass es bei Anwendung in einem Winkel von 45° zu der Achse der Tubusöffnung (20) angeordnet ist.

4. Vorrichtung nach einem der vorhergehenden Ansprüche, **dadurch gekennzeichnet, dass** das proximale Ende des Entnahmerohrs (22) wenigstens einen seitlich ausgebildeten Steg (32) aufweist.

5. Vorrichtung nach Anspruch 1, **dadurch gekennzeichnet, dass** das proximale Ende des Entnahmerohrs (22) einen dreieckigen Querschnitt aufweist.

6. Vorrichtung nach Anspruch 1, **dadurch gekennzeichnet, dass** der Querschnitt des proximalen Endes des Entnahmerohrs (22) mit wenigstens zwei radial symmetrisch angeordnete Kerben (25) ausgeführt ist.

7. Vorrichtung nach einem der vorhergehenden Ansprüche, **dadurch gekennzeichnet, dass** der Anschluß (12, 14) an das Patientenbeatmungssystem und der Tubusanschluss (16) in Form eines Y-Stückes ausgebildet sind.

8. Vorrichtung nach einem der vorhergehenden Ansprüche, **dadurch gekennzeichnet, dass** wenigstens ein Teil des Entnahmerohrs (22) aus einem elastischen Material ausgeführt ist.

9. Vorrichtung nach einem der vorhergehenden Ansprüche, **dadurch gekennzeichnet, dass** das Entnahmerohr (22) beweglich in der Wandöffnung (24) des Tubusanschlussstückes (10) befestigt ist, vorzugsweise mit einer elastischen Membran (36).

10. Vorrichtung nach einem der Ansprüche 1 bis 8, **dadurch gekennzeichnet, dass** das Entnahmerohr (22) mit einem Federelement (38) in der Wandöffnung (24) des Tubusanschlussstückes (10) befestigt ist, wobei vorzugsweise wenigstens ein O-Ring (40) zur pneumatischen Dichtung vorgesehen ist.

11. System mit einer Vorrichtung nach einem der Ansprüche 1-10 und einem Tubus (18) unit einer Tubusöffnung (20), **dadurch gekennzeichnet, dass** ein Außenmaß des Entnahmerohres (22) größer als ein Durchmesser der Tubusöffnung (20) ist.

12. System nach Anspruch 11, **dadurch gekennzeichnet, dass** bei einem Zusammenwirken des Entnahmerohrs (22) mit dem Tubus (18) die Tubusöffnung (20) maximal um 30 % verringert wird.

## Claims

1. A device for obtaining a breathing gas sample, comprising at least:
a tube body connection piece (10) having a wall opening (24), a connection (12, 14) to a patient breathing system, a tube body connection (16), and a withdrawal tube (22) extending from the wall opening (24) in the direction of the tube body connection (16),
**characterized in that**
the proximal end of the withdrawal tube (22) comprises at least one recess clearance (23) such that a breathing gas flow perpendicular to the breathing gas withdrawal direction is made possible.

2. A device according to claim 1, **characterized in that** the withdrawal tube (22) is arranged such that it is disposed centrically on the axis of the tube body opening (20), when in use.

3. A device according to claim 1, **characterized in that** the withdrawal tube (22) is arranged such that it is disposed at an angle of 45° with respect to the axis of the tube opening (20), when in use.

4. A device according to any of the preceding claims, **characterized in that** the proximal end of the withdrawal tube (22) comprises at least one laterally formed bar (32).

5. A device according to claim 1, **characterized in that** the proximal end of the withdrawal tube (22) comprises a triangular cross-section.

6. A device according to claim 1, **characterized in that** the cross-section of the proximal end of the withdrawal tube (22) is formed with at least two notches (25) disposed radial symmetrically.

7. A device according to any of the preceding claims, **characterized in that** the connection (12, 14) to the patient breathing system and the tube body connection (16) is formed in the form of a Y-piece.

8. A device according to any of the preceding claims, **characterized in that** at least part of the withdrawal tube (22) is made of an elastic material.

9. A device according to any of the preceding claims, **characterized in that** the withdrawal tube (22) is moveably mounted in the wall opening (24) of the tube body connection piece (10), preferably by an elastic membrane (36).

10. A device according to any of the claims 1 to 8, **characterized in that** the withdrawal tube (22) is mounted with a spring element (38) in the wall opening (24) of the tube body connection piece (10), wherein preferably at least one O-ring (40) for pneumatic sealing is provided.

11. A system comprising a device according to one of the claims 1 to 10 and a tube body (18) having a tube body opening (20), **characterized in that** an outer dimension of the withdrawal tube (22) is larger than the diameter of the tube opening (20).

12. A system according to claim 11, **characterized in that**, when the withdrawal tube (22) cooperates with the tube body (18), the tube body opening (22) is decreased by a maximum of 30%.

## Revendications

1. Dispositif de prélèvement d'un échantillon de gaz respiratoire,
comprenant au moins
une pièce de raccordement tubulaire (10) ayant une ouverture dans sa paroi (24), un raccord (12, 14) pour un système de respiration du patient, un raccord tubulaire (16) et ayant une canule de prélèvement (22) s'étendant depuis l'ouverture dans la paroi (24) en direction du raccord tubulaire (16),
**caractérisé**
**en ce que** l'extrémité proximale de la canule de prélèvement (22) comprend au moins un évidement (23) de telle sorte qu'une circulation du gaz respiratoire perpendiculairement à une direction de prélèvement du gaz respiratoire est rendue possible.

2. Dispositif selon la revendication 1, **caractérisé en ce que** la canule de prélèvement (22) est réalisée de telle sorte que lors de l'utilisation, elle est agencée en position centrale dans l'axe de l'ouverture du tube (20).

3. Dispositif selon la revendication 1, **caractérisé en ce que** la canule de prélèvement (22) est réalisée de telle sorte que lors de l'utilisation, elle est agencée dans un angle de 45° par rapport à l'axe de l'ouverture du tube (20).

4. Dispositif selon l'une des revendications précédentes, **caractérisé en ce que** l'extrémité proximale de la canule de prélèvement (22) comprend au moins une tige (32) réalisée en position latérale.

5. Dispositif selon la revendication 1, **caractérisé en ce que** l'extrémité proximale de la canule de prélèvement (22) présente une section triangulaire.

6. Dispositif selon la revendication 1, **caractérisé en ce que** la section de l'extrémité proximale de la canule de prélèvement (22) est réalisée avec au moins deux entailles (25) agencées en symétrie radiale.

7. Dispositif selon l'une des revendications précédentes, **caractérisé en ce que** le raccord (12, 14) pour le système de respiration du patient et le raccord tubulaire (16) sont réalisés sous la forme d'une pièce en Y.

8. Dispositif selon l'une des revendications précédentes, **caractérisé en ce qu'**au moins une partie de la canule de prélèvement (22) est fabriquée dans un matériau élastique.

9. Dispositif selon l'une des revendications précédentes, **caractérisé en ce que** la canule de prélèvement (22) est fixée de façon mobile dans l'ouverture de la paroi (24) de la pièce de raccordement tubulaire (10), de préférence avec une membrane élastique (36).

10. Dispositif selon l'une des revendications 1 à 8, **caractérisé en ce que** la canule de prélèvement (22) est fixée dans l'ouverture de la paroi (24) de la pièce de raccordement tubulaire (10) avec un élément de ressort (38), étant entendu qu'au moins un joint torique (40) est de préférence prévu aux fins de l'étanchéité pneumatique.

11. Système comprenant un dispositif selon l'une des revendications 1 à 10 et un tube (18) ayant une ouverture de tube (20), **caractérisé en ce qu'**une dimension extérieure de la canule de prélèvement (22) est supérieure à un diamètre de l'ouverture du tube (20).

12. Système selon la revendication 11, **caractérisé en ce que** lors d'une interaction de la canule de prélèvement (22) avec le tube (18), l'ouverture du tube (20) est réduite au maximum de 30 %.
